# EUROPEAN PATENT SPECIFICATION

(11) **EP 3 409 760 B1**
(45) Date of publication and mention of the grant of the patent: **19.07.2023**
(21) Application number: 18172521.9
(22) Date of filing: 16.05.2018
(51) Int. Cl.: C12M 1/12, C12N 5/00, G01N 33/50, C12M 1/00

(54) **METHOD FOR CONTROLLING THE ANALYSIS OF A BIOLOGICAL MATERIAL USING A PSEUDO TISSUE.**
VERFAHREN ZUR KONTROLLE DER ANALYSE EINES BIOLOGISCHEN MATERIALS UNTER VERWENDUNG EINES PSEUDO-GEWEBES.
MÉTHODE POUR CONTRÔLER L'ANALYSE D'UN MATÉRIAU BIOLOGIQUE EN UTILISANT UN PSEUDO-TISSU.

(30) Priority: 16.05.2017 JP 2017097365
(43) Date of publication of application: 05.12.2018
(73) Proprietor: SYSMEX CORPORATION, Kobe-shi Hyogo 651-0073 (JP)
(72) Inventor: Kiriyama, Maria, Kobe-shi, Hyogo, 651-0073 (JP); Yamada, Kohei, Kobe-shi, Hyogo, 651-0073 (JP)
(74) Representative: De Clercq & Partners

(56) References cited:
- EP-A1- 2 404 992
- EP-A1- 2 799 093
- EP-A1- 2 949 747
- WO-A1-2005/012512
- WO-A1-2012/131000

## Description

### TECHNICAL FIELD

The present invention relates to a method for controlling accuracy of specimen analysis using a fixed embedded pseudo tissue.

### BACKGROUND

Tissue specimens such as lesion tissues collected from a living body are specimens that can be used to obtain useful information for discrimination of diseases and understanding of medical conditions. However, a tissue specimen cannot be stored for a long time as it is. Usually, tissue specimens are fixed with a fixing agent such as formalin and embedded in an embedding agent such as paraffin for long-term preservation. Thereafter, the fixed and embedded tissue specimens can be cut out as necessary, and desired measurement and analysis can be performed on the obtained sections. For example, in histopathological examination, a thin sliced section of a fixed and embedded tissue specimen is attached to a glass slide, the embedding agent is removed and the tissue is stained, and pathological diagnosis is performed by microscopic observation.

Quantitative analysis of tissue specimens at the cell level has also been performed. The embedding agent is removed from the fixed and embedded tissue specimen, the tissue is treated with an enzyme or the like, and the obtained cells are analyzed to obtain information such as cell morphology and expression of molecular markers in the cells. For example, in US 2015/0,276,574 A, pathological tissues from which the embedding agent has been removed are enzymatically treated and dispersed into individual cells, and the obtained cells are analyzed by flow cytometry.

On the other hand, in order to verify the inspection results by tissue observation and to control accuracy of cell measurement, it is necessary to also subject a positive control and a negative control to inspection and measurement in parallel with the specimen to be measured. For example, US 2003/0,124,563 A discloses using a pseudo tissue prepared from cells of a cell line as a control for histopathological examination. The pseudo tissue of US 2003/0,124,563 A is a cell mass adhered with fibrin, prepared by adding fibrinogen and thrombin to the cells.

### SUMMARY

When cells contained in fixed and embedded tissue specimens are analyzed with a flow cytometer, at least the embedding agent is removed and the tissue specimens are dispersed into the cells. However, the pseudo tissue of US 2003/0,124,563 A is a cell mass formed by adhering cells with fibrin. Therefore, the pseudo tissue of US 2003/0,124,563 A cannot be dispersed into individual cells by the enzyme normally used for tissue treatment. Plasmin is used for degradation of fibrin, but tissues are not dispersed into individual cells by plasmin. Therefore, the pseudo tissue of US 2003/0,124,563 A is not suitable for control of cell analysis by a flow cytometer. Therefore, it is desirable to use a pseudo tissue sample as a control for cell analysis by flow cytometer.

The present invention provides a method for controlling accuracy of specimen analysis including
a first step of dispersing the cells of the fixed embedded pseudo tissue comprising a plurality of cells derived from a cell line and a cell adhesion molecule produced by the cells, wherein the cells are adhered by the cell adhesion molecule in the pseudo tissue, into individual cells to prepare a control sample, and obtaining a measured value representing the number of cells by flow cytometer,
a second step of dispersing cells contained in a fixed embedded tissue specimen into individual cells to prepare a measurement sample, and obtaining a measured value representing the number of cells by flow cytometer, and
a step of comparing the measured value of the control sample with a predetermined reference value and determining whether or not the second step has been appropriately performed based on the comparison result.

In particular embodiments, the method further comprises a step of obtaining a measured value representing the number of molecular marker-positive cells, in the measurement step by the flow cytometer.

In particular embodiments, in the determination step, when the difference or the ratio between the measured value of the control sample and the predetermined reference value is within a predetermined numerical range, it is determined that the second step has been appropriately performed, and
when the difference or the ratio between the measured value of the control sample and the predetermined reference value deviates from the predetermined numerical range, it is determined that the second step has not been appropriately performed.

In particular embodiments, in the first step, a pseudo tissue is acquired from the fixed embedded pseudo tissue, a protease is brought into contact with the pseudo tissue, and the cell adhesion molecule in the pseudo tissue is degraded, whereby the cells constituting the pseudo tissue are dispersed into individual cells, and
in the second step, a tissue specimen is acquired from the fixed embedded tissue specimen, a protease of the same type as the protease is brought into contact with the tissue specimen, and the cell adhesion molecule in the tissue specimen is degraded, whereby the cells constituting the tissue specimen are dispersed into individual cells.

The present invention provides a method for controlling accuracy of specimen analysis comprising
a first step of acquiring a pseudo tissue from the fixed embedded pseudo tissue comprising a pseudo tissue comprising a plurality of cells derived from a cell line and a cell adhesion molecule produced by the cells, wherein the cells are adhered by the cell adhesion molecule in the pseudo tissue, and obtaining a measured value representing an expression level of a molecular marker or the number of molecular marker-positive cells, from the pseudo tissue, and
a step of comparing the measured value with a predetermined reference value and determining whether or not the first step has been appropriately performed based on the comparison result.

In particular embodiments, the step of obtaining the measured value is performed by flow cytometer or a microscope.

In particular embodiments, in the determination step, when the difference or the ratio between the measured value of the pseudo tissue and the predetermined reference value is within a predetermined numerical range, it is determined that the first step has been appropriately performed, and
when the difference or the ratio between the measured value of the pseudo tissue and the predetermined reference value deviates from the predetermined numerical range, it is determined that the first step has not been appropriately performed.

In particular embodiments, the method further comprises a second step of acquiring the tissue specimen from a fixed embedded tissue specimen, and obtaining a measured value representing an expression level of a molecular marker or the number of molecular marker-positive cells, from the tissue specimen.

### BRIEF DESCRIPTION OF THE DRAWINGS

Fig. 1A is a photograph showing a fixation step of a cell mass containing cells of two types of cell lines.
Fig. 1B is a photograph showing a columnar cell mass (pseudo tissue) prepared in Example 1.
Fig. 1C is a photograph showing a paraffin block of a formalin-fixed paraffin-embedded (FFPE) pseudo tissue prepared in Example 1.
Fig. 2A is a photograph showing a fixation step of a cell mass containing cells of one type of cell line.
Fig. 2B is a photograph showing a pseudo tissue prepared in Example 2.
Fig. 3 provides photographs of a thin sliced section of an FFPE pseudo tissue stained with hematoxylin/eosin (HE) as observed with a microscope.
Fig. 4A is a schematic diagram showing cutting positions of an FFPE pseudo tissue.
Fig. 4B provides photographs of thinly sliced sections obtained by cutting an FFPE pseudo tissue in the upper part, the middle part and the lower part and staining with HE, as observed with a microscope.
Fig. 5 provides photographs of pseudo tissues obtained by deparaffinizing thin sliced sections of FFPE pseudo tissues.
Fig. 6A provides photographs of a pseudo tissue obtained by deparaffinizing thinly sliced section of an FFPE pseudo tissue and staining immunohistologically, as observed with a microscope.
Fig. 6B is a graph showing ratios of cells expressing estrogen receptor (ER) to cells not expressing ER.
Fig. 6C is a graph showing ratios of cells expressing progesterone receptor (PgR) to cells not expressing PgR.
Fig. 7A is a graph showing cell recovery rates when a pseudo tissue obtained by deparaffinizing a thinly sliced section of an FFPE pseudo tissue was dispersed into individual cells by Dispase I + II and the obtained cells were measured with a flow cytometer.
Fig. 7B is a graph showing cell recovery rates when a pseudo tissue obtained by deparaffinizing a thinly sliced section of an FFPE pseudo tissue, was dispersed into individual cells by Dispase II and the obtained cells were measured with a flow cytometer.
Fig. 8A is a histogram of molecular marker-positive cells detected by flow cytometer. In this histogram, the horizontal axis is fluorescence intensity, and the vertical axis is counts (the number of cells).
Fig. 8B is a histogram of molecular marker-positive cells detected by flow cytometer. In this histogram, the horizontal axis is fluorescence intensity, and the vertical axis is counts (the number of cells).
Fig. 9A provides photographs of cells immunostained with an anti-Ki-67 antibody and a fluorescently labeled secondary antibody taken by an imaging flow cytometer.
Fig. 9B provides photographs of cells immunostained with an anti-Ki-67 antibody and a fluorescently labeled secondary antibody taken by an imaging flow cytometer.
Fig. 9C provides photographs of cells reacted with only a fluorescently labeled secondary antibody taken by an imaging flow cytometer.
Fig. 10 provides photographs showing expression of cell adhesion molecules in cells of various cell lines.
Fig. 11 provides photographs of a pseudo tissue obtained by deparaffinizing a thin sliced section of an FFPE pseudo tissue, which was then immunostained with an anti-E-cadherin antibody and observed with a fluorescence microscope.

### DETAILED DESCRIPTION OF THE PREFERRED EMBODIMENTS

### [1. Fixed Embedded Pseudo Tissue]

Disclosed but not claimed herein is a pseudo tissue fixed by a fixing agent and embedded in an embedding agent. The pseudo tissue is a cell mass that imitates a tissue collected from a living body, and includes a plurality of cells derived from a cell line and a cell adhesion molecule produced by the cells. The number of cells is determined in consideration of the type of the tissue specimen and the method of obtaining the measured value to be described later and the like. In the pseudo tissue, cells are adhered to each other by the cell adhesion molecule, thereby forming a tissue-like structure. In a fixed embedded pseudo tissue, intracellular and intercellular moisture is replaced with an embedding agent.

It is preferable that the pseudo tissue included in the fixed embedded pseudo tissue is substantially homogeneous. The phrase "substantially homogeneous" refers to a state in which there is almost no deviation of cells and the like depending on the location in the pseudo tissue, and the cells are almost uniformly dispersed. For example, when the type and number of cells are unevenly distributed in any cut surface of the pseudo tissue, it is not suitable for accuracy control as envisaged herein, and cannot be said to be "substantially homogeneous".

The fixed embedded pseudo tissue can be handled in the same manner as a fixed and embedded tissue specimen generally prepared. That is, , the fixed embedded pseudo tissue can be cut with a microtome or the like to obtain a thin sliced section. A pseudo tissue can be acquired by removing the embedding agent from a thinly sliced section of the obtained fixed embedded pseudo tissue. On the obtained pseudo tissue, the same measurement and analysis can be performed as on the target tissue specimen. As described above, the fixed embedded pseudo tissue is suitable as a control sample for accuracy control of measurement and analysis, since it is possible to perform the steps from the preparation to the measurement of the measurement sample, in the same manner as the fixed and embedded tissue specimen.

The cells contained in the fixed embedded pseudo tissue are not particularly limited as long as they are cells derived from an established cell line and capable of producing a cell adhesion molecule. As such a cell line, a cell line derived from an epithelial cell is preferable, and a cell line of a tumor cell derived from an epithelial cell is more preferable. Examples include HCC1806, MBA468, BT20, AU565, BT474, BT483, BT549, CAMA1, HCC1419, HCC1428, HCC1500, HCC202, HCC1569, HCC1937, HCC1954, Hs578T, MDA-MB-157, MDA-MB-231, MDA-MB-361, MDA-MB-415, MDA-MB-435S, MDA-MB-436, MDA-MB-453, MDA-MB-468, SK-BR-3, T-47D, UACC812, UACC893, ZR-75-1, ZR-75-30, MCF10A, and the like.

The cell adhesion molecule is preferably a cell-cell adhesion factor, and examples thereof include cadherin and the like. However, fibrinogen and fibrin are not included within the term cell adhesion molecule referred to herein. Accordingly, the cells contained in the fixed embedded pseudo tissue are substantially not adhered by fibrin. In a biological tissue, cells are not adhered to each other by fibrinogen or fibrin, but are adhered to each other by an adhesion factor such as cadherin. In the pseudo tissue disclosed but not claimed herein, cells are adhered to each other by a cell adhesion molecule such as cadherin, not fibrinogen or fibrin, so the pseudo tissue is closer to a tissue collected from a living body. Therefore, similar pretreatment can be applied to the fixed embedded tissue specimen and the fixed embedded pseudo tissue of this embodiment, which is more suitable for accuracy control. As detailed further herein the fixed embedded pseudo tissue or the dispersed cell compositions obtained therefrom preferably does not comprise added fibrinogen, thrombin or fibrin, i.e. fibrinogen, thrombin or fibrin which is not produced by the cells themselves. The cells may be cells which do produce a cell-adhesion factor, but do not produce fibrinogen, thrombin or fibrin, more particularly the cells do not produce fibrinogen, thrombin and fibrin in an amount sufficient to adhere the cells.

The ability to produce a cell adhesion molecule can be easily confirmed by a method known in the art. For example, cells can be solubilized with a surfactant or the like, and whether or not a cell adhesion molecule is contained in the obtained supernatant can be confirmed by immunological methods such as Western blotting. In addition, expression of cell adhesion molecules in the cells may be confirmed by immunostaining the cells with an antibody that specifically binds to the cell adhesion molecule.

In the art, a tissue specimen to be measured expresses a predetermined molecular marker, and the tissue specimen is sometimes analyzed based on the detection of the molecular marker. It is preferable that the fixed embedded pseudo tissue disclosed herein can also be analyzed in the same manner as the tissue specimen. Therefore, the cells contained in the fixed embedded pseudo tissue may express the predetermined molecular marker. The predetermined molecular marker may be a molecule originally expressed in the cell or may be a molecule expressed in the cell by an operation such as gene introduction or stimulation.

In this context, the type of the molecular marker is not particularly limited, but examples thereof include membrane proteins, cytoplasmic proteins, and nuclear proteins. Among them, membrane proteins are preferred. The function of the molecular marker is not particularly limited, and the molecular marker may be, for example, a membrane receptor, a cell proliferation-related molecule, or the like. Examples of the molecular marker include estrogen receptor, progesterone receptor, Ki-67 and HER2, and the like.

The cells contained in the fixed embedded pseudo tissue may be cells derived from one type of cell line, but may be cells derived from two kinds of cell lines. That is, the fixed embedded pseudo tissue may include two types, a plurality of first cells derived from a first cell line and a plurality of second cells derived from a second cell line. By combining cells of two types of cell lines with different properties, it is possible to obtain a fixed embedded pseudo tissue with the properties of both cell lines. For example, the first cell line may be a cell line having a higher ability to produce a cell adhesion molecule than the second cell line. In this case, the second cell line may be a cell line that cannot form a pseudo tissue alone due to its low adhesiveness. In addition, the second cell line may be a cell line expressing the predetermined molecular marker. In this case, the first cell line may be a cell line that does not express the predetermined marker. By combining the cells of the first cell line with the cells of the second cell line as above, a fixed embedded pseudo tissue containing a certain proportion of cells expressing the predetermined molecular marker can be obtained. Accordingly, the fixed embedded pseudo tissue may comprise cells from two different cell types.

The number of first cells and the number of second cells in the fixed embedded pseudo tissue are determined in consideration of the type of the tissue specimen and the method of obtaining the measured value to be described later and the like. From the viewpoint of cell mass formation, in the case where the first cell line is a cell line having a higher ability to produce a cell adhesion molecule than the second cell line, the number of first cells in the fixed embedded pseudo tissue is preferably not less than the number of second cells. Cell-cell adhesion can be adjusted by adjusting the number of cells having high ability to produce a cell adhesion molecule. As a result, the formed cell mass can imitate a tissue specimen in terms of strength, elasticity and the like. Optionally, the number of first cells in the fixed embedded pseudo tissue is not less than the number of second cells and not more than 4 times the number of second cells.

As described above, in the fixed embedded pseudo tissue, the pseudo tissue is preferably substantially homogeneous. It is preferable that, in the fixed embedded pseudo tissue including the first cells and the second cells, the first cells and the second cells are not unevenly distributed irrespective of cleavage at any site, and the ratio of the number of first cell to the number of second cells on the cut surface is substantially the same. Also, preferably, the total number of the first cells and the second cells is uniform on any of the cut surfaces.

Although the shape and size of the fixed embedded pseudo tissue are not particularly limited, it is preferable that it has a shape and size that allows cut out of a thinly sliced section with a microtome or the like. The number of cells in the fixed embedded pseudo tissue is not particularly limited and may be any number as long as a pseudo tissue having a desired size can be obtained. For example, when the shape of a fixed embedded pseudo tissue is cylindrical with a base area of about 0.35 cm² (corresponding to the culture area per well of a standard 96-well plate), the fixed embedded pseudo tissue contains usually 2 × 10⁷ cells, and preferably 1 × 10⁷ cells.

The fixed embedded pseudo tissue can be prepared by fixing the above pseudo tissue with a fixing agent and embedding the fixed pseudo tissue with an embedding agent. The fixing agent is not particularly limited and can be appropriately selected from known fixing agents used for fixing cells or tissues in the art. Examples of such fixing agents include formalin (formaldehyde), glutaraldehyde, osmium tetroxide, acetic acid, lower alcohols, acetone, picric acid, chloroform, potassium dichromate, mercuric chloride, and the like. Examples of the lower alcohol include alcohols having 1 to 6 carbon atoms, and methanol or ethanol is preferable. Commercially available fixing agents may also be used. The fixing agent may be used alone or in combination of two or more.

The embedding agent is not particularly limited, and can be appropriately selected from known embedding agents used for preparing a tissue sample in the art. Examples of such embedding agents include paraffin, paraffin derivatives, celloidin, carbowax, agarose, non-heparinized serum, collagen, cellulose derivatives, chitin derivatives and chitosan derivatives. Commercially available embedding agents may also be used. The embedding agents may be used alone or in combination of two or more.

Among the above embedding agents, paraffin is particularly preferred. Paraffin is commonly used for preparing a tissue sample, and a method for removing paraffin from a paraffin-embedded tissue (deparaffinization treatment) has also been established. Therefore, the fixed embedded pseudo tissue in which the pseudo tissue is embedded with paraffin is particularly preferable.

In the art, the tissue specimen obtained by removing the embedding agent from the tissue sample is sometimes dispersed into individual cells by enzyme treatment or the like and analyzed at the cell level. In the fixed embedded pseudo tissue , since the cells are adhered to each other by the cell adhesion molecules in the same manner as the tissue specimen, the same analysis can be performed. That is, the pseudo tissue acquired by removing the embedding agent from the fixed embedded pseudo tissue can be dispersed into individual cells by treatment with a protease. The cells obtained from the pseudo tissue in this manner can be analyzed by flow cytometer or the like.

The protease for use as described herein is not particularly limited as long as it is an enzyme capable of eliminating cell-cell adhesion by digesting the cell adhesion molecules. As such protease, a protease capable of degrading cadherin is preferable. Examples of the protease include Dispase (trademark), collagenase, hyaluronidase, elastase, papain, trypsin, and the like. Dispase is a metalloprotease derived from Paenibacillus sp. and is commercially available.

### [2. Method for Preparing Fixed Embedded Pseudo Tissue]

A method for preparing the above fixed embedded pseudo tissue (hereinafter also simply referred to as "preparation method") is described but not claimed herein. In the preparation method provided herein, first, a plurality of cells derived from a cell line is incubated. During the incubation, the cells are adhered to each other by the cell adhesion molecule, and a cell mass having a tissue-like structure is formed. This cell mass is the pseudo tissue referred to herein.

Details of cell lines and cells derived therefrom are as described above. The conditions (temperature and atmosphere) and medium of the incubation are not particularly limited, and can be appropriately determined from known culture conditions and medium according to the type of cells. The culture vessel is not particularly limited, and the shape of the obtained pseudo tissue depends on the culture vessel. A multi-well plate such as a 96-well plate is preferable. Also, a cell migration assay plate such as Transwell (registered trademark) 96-wellplate may be used. In each well of the cell migration assay plate, the compartment containing cells and the compartment containing medium are separated by an insert having a membrane, so that it is convenient for the fixation and the recovery of the cell mass as described later.

Typically a predetermined number of cells are placed in a culture vessel together with the medium and incubated. The number of cells is as described above. The incubation time may be a time sufficient for the cells to adhere to each other by the cell adhesion molecule. Such incubation time is, for example, from 0.5 hour to 6 hours, preferably from 1.2 hours to 2 hours, and particularly preferably 1.5 hours. After the incubation, the formed pseudo tissue may be washed with a suitable buffer solution such as phosphate buffered saline (PBS). Preferably, care is taken that the pseudo tissue is not dispersed but that the cells remain adhered to each other.

It is preferable to properly culture cells derived from the cell line in advance before performing the above incubation. By recovering and counting the cultured cells, it is possible to prepare the number of cells necessary for the pseudo tissue. The time point at which to recover the cells is not particularly limited, and is preferably in the logarithmic growth phase. As a method for recovering the cells, a method capable of recovering the cells without damaging them is preferable. Examples thereof include a method in which cells are exfoliated from a culture vessel using trypsin or EDTA. Cells may be directly exfoliated from a culture vessel with a cell scraper or the like. The recovered cells are preferably washed with an appropriate buffer solution such as PBS, a serum-free medium, or the like. As necessary, dead cells and cell debris may be removed by density centrifugation or the like.

The cells contained in the fixed embedded pseudo tissue may be cells derived from one type of cell line, but may be cells derived from two kinds of cell lines. In the case of preparing a pseudo tissue including a plurality of first cells derived from a first cell line and a plurality of second cells derived from a second cell line, the first cells and the second cells are separately cultured in advance. Then, the cells of each cell line are recovered, and the number of cells is counted. Then, the first cell and the second cell are mixed at a predetermined ratio to obtain a cell suspension. The obtained cell suspension is placed in a culture vessel and incubated in the same manner as described above.

As described above, when the first cell line is a cell line having a higher ability to produce a cell adhesion molecule than the second cell line, it is preferable to mix them so that the number of first cells is not less than the number of second cells, and it is more preferable to mix them so that the number of first cells is not less than the number of second cells and not more than 4 times the number of second cells.

In the preparation method disclosed but not claimed herein, the formed pseudo tissue is fixed by a fixing agent. The type of fixing agent is as described above. It is preferable that the fixing agent is a liquid. When the fixing agent is powder or crystal, it is preferable to use a solution thereof. Hereinafter, the liquid fixing agent is also referred to as "fixing liquid". The fixation of cells is performed by removing the medium from the culture vessel containing the formed pseudo tissue and adding a fixing liquid to the pseudo tissue. The amount of the fixing liquid to be added is not particularly limited and may be an amount sufficient for fixing the tissue. The fixing time can be appropriately determined according to the type of fixing agent. For example, when formalin is used, it is preferable to fix the pseudo tissue for about 24 hours.

In the preparation method disclosed but not claimed herein, the fixed pseudo tissue is recovered from the culture vessel in order to perform the embedding described later. The recovered pseudo tissue may be washed with an appropriate buffer solution such as PBS. Alternatively, the recovered pseudo tissue may be dehydrated by immersion in a mixture of a lower alcohol (e.g., ethanol) and water.

In the preparation method disclosed but not claimed herein, the fixed pseudo tissue is embedded with an embedding agent. The type of embedding agent is as described above. By embedding, intracellular and intercellular moisture in the pseudo tissue is replaced with an embedding agent. The embedding method can be appropriately selected from publicly known methods depending on the type of the embedding agent to be used.

As an example of a procedure for embedding the fixed pseudo tissue, paraffin embedding will be described. Paraffin is water insoluble, thus does not penetrate a pseudo tissue containing moisture. Therefore, the pseudo tissue is dehydrated with a lower alcohol, preferably ethanol. Preferably, the pseudo tissue is dehydrated stepwise by being immersed in a rising series of ethanol. The rising series of ethanol is, for example, a series of ethanol solutions in which the ethanol content is increased stepwise to 70 v/v%, 80 v/v%, 90 v/v%, and 100 v/v%. Next, the dehydrated pseudo tissue is immersed in an organic solvent having affinity to paraffin to replace the lower alcohol in the pseudo tissue with the organic solvent. Examples of such organic solvent include xylene, benzene, toluene, and the like. Among them, xylene is preferable. Then, the pseudo tissue containing the organic solvent is immersed in the molten paraffin to replace the organic solvent in the pseudo tissue with paraffin. After cooling, a fixed embedded pseudo tissue embedded in paraffin is obtained. The method can be a method for preparing dispersed cells from a fixed pseudo tissue, said method comprises the steps for preparing a fixed embedded pseudo tissue as described above, and further the steps of removal of the embedding agent, and dispersal of the pseudo tissue into individual cells by a protease as described elsewhere herein.

In the preparation method disclosed but not claimed herein, fibrinogen, thrombin and fibrin are not substantially added to the pseudo tissue during the formation of the above cell mass (pseudo tissue). The phrase "not substantially added" refers that fibrinogen, thrombin and fibrin are not added to cells, for the purpose of adhesion of fibrin-mediated cells and formation of pseudo tissues in the preparation method. However, the cases where fibrinogen, thrombin and fibrin are added in such an amount that no pseudo tissue is formed via fibrin, such as the case where trace amounts of fibrinogen, thrombin or fibrin are contained in the medium, are excluded. Accordingly, the in preparation methods provided herein, fibrinogen, thrombin or fibrin are not added per se or as a substantial ingredient of medium or any other reagent. Preferably, fibrinogen, thrombin and fibrin are not substantially added to the pseudo tissue in the formation step and fixation step of the cell mass (pseudo tissue). Optionally, fibrinogen, thrombin and fibrin are not substantially added to the pseudo tissue in the formation step, fixation step and embedding step of the cell mass (pseudo tissue). Preferably, fibrinogen, thrombin and fibrin are not added in any step.

### [3. Method for Controlling Accuracy of Specimen Analysis]

### (Accuracy control of cell analysis by flow cytometer)

The scope of the present disclosure includes a method for controlling accuracy of specimen analysis, using the above fixed embedded pseudo tissue (hereinafter also referred to simply as "accuracy control method"). As a particular embodiment, a method for controlling accuracy of cell analysis by flow cytometer (FCM) will be described below.

The accuracy control method provided herein is suitable for controlling analysis accuracy when an embedding agent is removed from a fixed and embedded tissue specimen (hereinafter also referred to as "fixed embedded tissue specimen") to acquire a tissue specimen, and the acquired tissue specimen is dispersed into individual cells and analyzed by FCM. In the accuracy control method provided herein, as a first step, a control sample is prepared from the fixed embedded pseudo tissue, and the control sample is measured with a flow cytometer to obtain a measured value representing the number of cells. Hereinafter, the measured value obtained by measurement of the control sample is also referred to as "measured value of the control sample". As a second step, a measurement sample is prepared from a fixed embedded tissue specimen, and the measurement specimen is measured with a flow cytometer to obtain a measured value representing the number of cells. Hereinafter, the measured value obtained by measurement of the measurement sample is also referred to as "measured value of the measurement sample".

In the accuracy control method provided herein, it is preferable to perform the second step in parallel with the first step, for more precise accuracy control. For example, preparation of a control sample from the fixed embedded pseudo tissue and preparation of a measurement sample from the fixed embedded tissue specimen may be performed substantially simultaneously or sequentially. In addition, the acquisition of the measured value of the control sample and the acquisition of the measured value of the measurement sample may be continuously performed by FCM.

In the accuracy control method provided herein, the tissue specimen is not particularly limited as long as it is a tissue collected from a living body by surgery, biopsy or the like. Such tissue specimen may be any of a tissue of a solid tumor (hereinafter referred to as "tumor tissue"), a non-neoplastic lesion tissue, and a normal tissue. The solid tumor is a malignant tumor other than blood tumors, including carcinomas, sarcomas, and lymphomas. The non-neoplastic lesion tissue is a lesion tissue other than tumors, and examples thereof include tissues in which polyps, inflammation, ulcers or the like occurred. The normal tissue is a tissue other than tumor tissues and non-neoplastic lesion tissues. In particular embodiments, a fixed embedded tissue specimen can be obtained by fixing and embedding the above tissue specimen by methods known in the art.

In the first step, first, a pseudo tissue is acquired from the fixed embedded pseudo tissue. In the second step, a tissue specimen is acquired from the fixed embedded tissue specimen. These steps are performed by removing the embedding agent from the fixed embedded pseudo tissue and the fixed embedded tissue specimen. Removal of the embedding agent can be performed, for example, with a reagent capable of dissolving or degrading the embedding agent. In this embodiment, it is preferable that the embedding agent of the fixed embedded pseudo tissue and the embedding agent of the fixed embedded tissue specimen are the same or the same type, for more precise accuracy control. The same type of embedding agent is, for example, an embedding agent that has the same main component and can be removed from the tissue by the same treatment. In a preferred embodiment, both fixed embedded pseudo tissue and fixed embedded tissue specimen are paraffin-embedded.

As an example of a procedure for removing the embedding agent, deparaffinization treatment of a paraffin-embedded tissue will be described below. The paraffin-embedded pseudo tissue and tissue specimen are each immersed in an organic solvent having affinity to paraffin. The paraffin is dissolved and removed by the organic solvent, and a pseudo tissue and a tissue specimen can be obtained. Such organic solvent is as described above. The treatment time and the treatment temperature in the deparaffinization treatment with the organic solvent can be appropriately decided according to the type of the tissue specimen and the like.

Since the organic solvent permeates the pseudo tissue and the tissue specimen obtained by the deparaffinization treatment, it is preferable to re-hydrophilize the pseudo tissue and the tissue specimen. The re-hydrophilization treatment is performed by immersing the pseudo tissue and the tissue specimen in a lower alcohol, preferably ethanol. In a preferred embodiment, the pseudo tissue and the tissue specimen are rehydrated stepwise by being immersed in a descending series of ethanol. The descending series of ethanol is, for example, a series of ethanol solutions in which the ethanol content is lowered stepwise to 100 v/v%, 90 v/v%, 80 v/v%, and 70 v/v%.

For the method described herein, it is sufficient to obtain an amount of cells sufficient for measurement with FCM from each of the fixed embedded pseudo tissue and the fixed embedded tissue specimen. Therefore, in the first step, the pseudo tissue may be acquired from the thin sliced section containing the fixed embedded pseudo tissue. In the second step, the tissue specimen may be acquired from the thin sliced section containing the fixed embedded tissue specimen. These thin sliced sections can be obtained by cutting the fixed embedded pseudo tissue and the fixed embedded tissue specimen with a microtome or the like.

In the first step, the obtained pseudo tissue is dispersed into individual cells to prepare a control sample containing the cells of the pseudo tissue. In the second step, the obtained tissue specimen is dispersed into individual cells to prepare a measurement sample containing cells of the tissue specimen. The dispersion of the pseudo tissue and the tissue specimen can be performed, for example, by degrading the cell adhesion molecule by bringing the pseudo tissue and the tissue specimen into contact with a protease. Specifically, the pseudo tissue and the tissue specimen are placed in separate vessels, a solution of protease is added to these vessels, and the vessels are incubated. After the pseudo tissue and the tissue specimen are dispersed into individual cells, the above control sample and the above measurement sample can be obtained by deactivating the protease. The treatment time and the treatment temperature in the protease treatment can be appropriately determined according to the types of protease and tissue specimen and the like. Details of the protease are as described above. In particular embodiments, it is preferable to treat the pseudo tissue and the tissue specimen with the same or the same type of protease, for more precise accuracy control. The same type of protease may be, for example, a protease having the activity of degrading the same cell adhesion molecule and having high identity (for example, a sequence identity of at least 80%, 90% or 95%) of the amino acid sequence.

In particular embodiments, measured values representing the number of cells in the control sample and the measurement sample are obtained by flow cytometer. The type of the flow cytometer is not particularly limited, and a commercially available flow cytometer can be used. In a particular embodiment, an imaging flow cytometer may be used. The imaging flow cytometer is a flow cytometer equipped with an imaging unit such as a camera and can acquire images of individual cells. By using the imaging flow cytometer, multiple parameters including cell size and aspect ratio can be obtained based on the image of the imaged cell.

In the measurement by FCM, it is preferable to irradiate individual cells in each sample with light to acquire optical information. The optical information includes scattered light information and fluorescence information. Specifically, first, a control sample is introduced into a flow cell of FCM, and when each cell in the sample passes through the flow cell, the cell is irradiated with light. Then, scattered light and fluorescence emitted from the cell are measured to acquire scattered light information and fluorescence information. Similarly for the measurement sample, scattered light information and fluorescence information are acquired. The order of measuring the control sample and the measurement sample is not particularly limited. In this embodiment, based on the optical information acquired by FCM, measured values representing the number of cells of the control sample and the number of cells of the measurement sample can be acquired. The measured value representing the number of cells may be the number of cells themselves or may be a cell concentration (for example, cells/mL).

Examples of the scattered light information include pulse peaks, pulse widths, pulse areas, scattered light intensities and the like of forward scattered light (for example, a light receiving angle of around 0 to 20 degrees) and side scattered light (a light receiving angle of around 90 degrees). In the art, it is known that side scattered light reflects internal information such as cell nuclei and granules, and forward scattered light reflects cell size. In a particular embodiment, it is preferable to acquire the forward scattered light intensity as the scattered light information. Examples of the fluorescence information include fluorescence intensity, fluorescence pulse width, fluorescence pulse area and the like, and fluorescence intensity is preferable. The wavelength of the excitation light to be irradiated can be appropriately selected according to the fluorescent substance.

The light source of the FCM is not particularly limited, and a light source having a wavelength suitable for exciting the fluorescent substance can be selected appropriately. As the light source, for example, a blue semiconductor laser, a red semiconductor laser, an argon laser, a He-Ne laser, a mercury arc lamp and the like are used.

In particular embodiments, expression of a predetermined molecular marker in a tissue specimen may be analyzed. That is, in measurement by FCM, a measured value representing the number of molecular marker-positive cells may be further obtained. The measured value representing the number of molecular marker-positive cells may be the number itself of molecular marker-positive cells or the concentration of molecular marker-positive cells (for example, cells/mL). Details of the molecular marker are as described above. The measurement of the molecular marker-positive cells may be performed, for example, by immunostaining the cells contained in the control sample and the measurement sample with an antibody (primary antibody) that specifically binds to the molecular marker. In this case, it is preferable that the primary antibody is labeled with a fluorescent substance. When an unlabeled primary antibody is used, an antibody (secondary antibody) labeled with a fluorescent substance that specifically binds to the primary antibody may be used. Examples of the fluorescent substance include fluorescent dyes such as fluorescein isothiocyanate (FITC), rhodamine and Alexa Fluor (registered trademark), fluorescent proteins such as GFP, and the like.

When cells immunostained with the antibody as described above are irradiated with light by FCM, a fluorescent signal is generated from the fluorescent substance labeled with the antibody. In particular embodiments, a fluorescence signal is measured by FCM, and based on the fluorescence information obtained from the signal, a measured value representing the number of molecular marker-positive cells can be acquired. For example, when the fluorescence information is the fluorescence intensity, a cell whose fluorescence intensity is higher than a predetermined value may be counted as a molecular marker-positive cell. The predetermined value regarding the fluorescence intensity can be appropriately determined.

In the case of immunostaining the cells contained in the control sample and the measurement sample, it is preferable to perform a treatment to activate the antigen in the pseudo tissue and the tissue specimen, after removal of the above embedding agent and re-hydrophilization treatment. The antigen activation treatment itself is known in the art and can be performed, for example, by using a heat treatment, an antigen activating agent or the like. Examples of the heat treatment include putting a pseudo tissue and a tissue specimen in a suitable buffer solution such as a citrate buffer solution or a TE buffer solution and incubating them at 95°C to 99°C. Examples of the antigen activating agent include Immunosaver (registered trademark) and the like.

In particular embodiments of the accuracy control method provided herein, the measured value of the control sample is compared with a predetermined reference value, and whether or not the second step has been appropriately performed is determined based on the comparison result. The composition of the fixed embedded pseudo tissue of this embodiment is constant, thus, when the preparation and measurement of the control sample have been appropriately performed, the obtained measured value is considered to converge to a substantially constant value. Therefore, it is possible to determine whether or not the second step has been appropriately performed, depending on the degree of difference between the measured value of the control sample and the predetermined reference value.

The numerical value itself of the predetermined reference value is not particularly limited and can be determined as appropriate. For example, a plurality of control samples may be prepared from the fixed embedded pseudo tissue of this embodiment and measured by FCM, and a statistical representative value of these measured values may be used as the predetermined reference value. Examples of the statistical representative value include an average value, a median value, and the like.

In particular embodiments, for example, when the difference or the ratio between the measured value of the control sample and the predetermined reference value is within the predetermined numerical range, it may be determined that the second step has been appropriately performed. Even when the above difference or ratio is the same as the numerical value at both ends of the predetermined numerical range, it may be determined that the second step has been appropriately performed. In this embodiment, the determination that the second step has been appropriately performed means that a series of operations from the preparation of the measurement sample to the acquisition of the measured value is appropriately performed. Therefore, the accuracy control method of this embodiment makes it possible to control the accuracy of the process from the preparation of the measurement sample from the fixed embedded tissue specimen to the measurement. The predetermined numerical range itself is not particularly limited and can be determined as appropriate.

On the other hand, when the difference or the ratio between the measured value of the control sample and the predetermined reference value deviates from the predetermined numerical range, it may be determined that the second step has not been appropriately performed. In this embodiment, the determination that the second step has not been appropriately performed means that all or a part of the series of operations from the preparation of the measurement sample to the acquisition of the measured value has not been appropriately performed.

In other embodiments of the accuracy control method, a control sample and a measurement sample are prepared in the same manner as described above. In this embodiment, before the acquisition of the measured value of the measurement sample, the acquisition of the measured value of the control sample is performed. It is possible to compare the measured value of the control sample with the predetermined reference value and to select whether or not to perform the acquisition of the measured value of the measurement sample based on the comparison result. For example, when the difference or the ratio between the measured value of the control sample and the predetermined reference value is within the predetermined range, the acquisition of the measured value of the measurement sample is performed. When the difference or the ratio between the measured value of the control sample and the predetermined reference value deviates from the predetermined range, the acquisition of the measured value of the measurement sample is not performed. Alternatively, when the difference or the ratio between the measured value of the control sample and the predetermined reference value deviates from the predetermined range, the user is notified thereof. The user can select whether or not to perform the acquisition of the measured value of the measurement sample while considering the notification.

### (Accuracy control of analysis of molecular markers)

As a further embodiment of methods of using the fixed embedded pseudo tissue as described herein, a method for controlling accuracy of analysis of an expression level of a molecular marker or the number of molecular marker-positive cells will be described below. In the accuracy control method provided herein, as a first step, a pseudo tissue is acquired from the above fixed embedded pseudo tissue, and a measured value representing an expression level of a molecular marker or the number of molecular marker-positive cells is obtained from the pseudo tissue. The pseudo tissue is acquired by removing the embedding agent from the fixed embedded pseudo tissue. Details of the embedding agent are as described above.

In the first step, the measured value representing the expression level of the molecular marker or the number of molecular marker-positive cells is obtained from the obtained pseudo tissue. The measured value can be obtained by measuring a sample prepared from the pseudo tissue. The type of the sample to be prepared may be appropriately determined according to the measuring means. Hereinafter, the measured value obtained by measurement of the sample prepared from the pseudo tissue is also referred to as "measured value of the pseudo tissue". The measured value representing the expression level of the molecular marker may be a protein level itself of the molecular marker in all or a part of the obtained pseudo tissue or may be a value reflecting the protein level.

The protein level of the molecular marker in all or a part of the obtained pseudo tissue can be obtained, for example, by measuring the protein level of the molecular marker in a supernatant obtained by solubilizing all or a part of the pseudo tissue. Specifically, the pseudo tissue obtained by removing the embedding agent is solubilized with a suitable surfactant, and the obtained supernatant is measured by an immunological technique using the above primary antibody (for example, Western blotting, ELISA method, or the like), it is possible to obtain a measured value representing the protein level of the molecular marker.

The value reflecting the protein level of the molecular marker in all or a part of the obtained pseudo tissue can be obtained by, for example, FCM. Specifically, the pseudo tissue obtained by removing the embedding agent is subjected to re-hydrophilization treatment and brought into contact with the protease in the same manner as described above, and the pseudo tissue is dispersed into individual cells. Then, all or a part of the obtained cells are immunostained using an antibody labeled with a fluorescent substance and measured by FCM as described above, so that fluorescence information corresponding to the value reflecting the protein level of the molecular marker can be acquired. Examples of such fluorescence information include the sum of the fluorescence intensities of cells having a fluorescence intensity equal to or higher than the predetermined value, in a histogram which has the number of cells on the vertical axis and the fluorescence intensity on the horizontal axis, and the like.

The measured value representing the number of molecular marker-positive cells can be obtained by, for example, FCM. Details of the acquisition of the measured value by FCM are as described above.

The measured value representing the number of molecular marker-positive cells may be the number itself of molecular marker-positive cells obtained by counting by microscopic observation. The number of molecular marker-positive cells can be counted, for example, by observing immunohistologically stained pseudo tissues with a microscope. Specifically, the above re-hydrophilization treatment is performed on the pseudo tissue obtained by removing the embedding agent, and the pseudo tissue is immunohistologically stained with the above primary antibody. As necessary, the above antigen activation treatment may be performed on the pseudo tissue after removal of the above embedding agent and re-hydrophilization treatment. It is preferable that the primary antibody is labeled with a fluorescent substance or an enzyme. When an unlabeled primary antibody is used, a fluorescent substance or a secondary antibody labeled with an enzyme may be used. When the pseudo tissue is immunohistologically stained with an antibody having a fluorescent substance, the molecular marker-positive cells can be detected and counted by observation with a fluorescence microscope. When the pseudo tissue is immunohistologically stained with an antibody having an enzyme, the molecular marker-positive cells can be observed with an optical microscope by allowing the pseudo tissue to react with a chromogenic substrate. As necessary, counterstaining with hematoxylin or the like may be performed on the pseudo tissue.

The fluorescent substance is as described above. Examples of the enzyme include alkaline phosphatases and peroxidases. The substrate of the enzyme can be appropriately selected from known substrates according to the type of the enzyme. When an alkaline phosphatase is used as the enzyme, a chromogenic substrate such as 5-bromo-4-chloro-3-indolyl phosphate (BCIP) or p-nitro blue tetrazolium (NBT) is used. When the enzyme is a peroxidase, a chromogenic substrate such as 3,3'-diaminobenzidine (DAB), 3-amino-9-ethylcarbazole (AEC), 2,2'-azinobis(3-ethylbenzothiazoline-6-sulfonic acid ammonium) (ABTS), 1,2-phenylenediamine (OPD) or 3,3',5,5'-tetramethylbenzidine (TMB) is used.

In the accuracy control method provided herein, the measured value of the pseudo tissue obtained as described above is compared with a predetermined reference value, and whether or not the first step has been appropriately performed is determined based on the comparison result. As described above, when the preparation and measurement of the samples are appropriately performed from the fixed embedded pseudo tissue of this embodiment, the obtained measured value is considered to converge to a substantially constant value. Therefore, it is possible to determine whether or not the first step has been appropriately performed, depending on the degree of difference between the measured value of the pseudo tissue and the predetermined reference value. As described above, according to the accuracy control method of this embodiment, it is possible to predict whether or not the series of operations from the preparation to the measurement of the sample can be appropriately performed, before performing the preparation and measurement of the sample of the fixed embedded tissue specimen. Details of the reference value are as described above.

In particular embodiments, for example, when the difference or the ratio between the measured value of the pseudo tissue and the predetermined reference value is within the predetermined numerical range, it may be determined that the first step has been appropriately performed. Even when the above difference or ratio is the same as the numerical value at both ends of the predetermined numerical range, it may be determined that the first step has been appropriately performed. In this embodiment, the determination that the first step has been appropriately performed means that a series of operations from the preparation of the sample from the fixed embedded pseudo tissue to the acquisition of the measured value has been appropriately performed. Based on this determination result, it may be determined to perform the preparation and measurement of the sample as in the first step on the fixed embedded tissue specimen. The predetermined numerical range itself is not particularly limited and can be determined as appropriate.

On the other hand, when the difference or the ratio between the measured value of the pseudo tissue and the predetermined reference value deviates from the predetermined numerical range, it may be determined that the first step has not been appropriately performed. In this embodiment, the determination that the first step has not been appropriately performed means that all or a part of the series of operations from the preparation of the sample from the fixed embedded pseudo tissue to the acquisition of the measured value has not been appropriately performed. Based on this determination result, it may be determined not to perform the preparation and measurement of the sample as in the first step on the fixed embedded tissue specimen. In this case, appropriate conditions can be studied by the preparation and measurement of the sample, by performing the accuracy control method of this embodiment again by adjusting the treatment and the reagent to be used in the preparation and measurement of the sample and the like.

In particular embodiments, preparation of a sample from a fixed embedded tissue specimen and measurement of the sample may be performed in parallel with the above first step. That is, the accuracy control method of this embodiment may further include a second step of acquiring a tissue specimen from a fixed embedded tissue specimen, and obtaining a measured value representing an expression level of a molecular marker or the number of molecular marker-positive cells, from the tissue specimen. Hereinafter, the measured value obtained by measurement of the sample prepared from the tissue specimen is also referred to as "measured value of the tissue specimen". In the case of performing the second step, the measured value of the pseudo tissue obtained in the first step is compared with the predetermined reference value, and whether or not the second step has been appropriately performed may be determined based on the comparison result.

In particular embodiments, for example, when the difference or the ratio between the measured value of the pseudo tissue and the predetermined reference value is within the predetermined numerical range, it may be determined that the second step has been appropriately performed. Even when the above difference or ratio is the same as the numerical value at both ends of the predetermined numerical range, it may be determined that the second step has been appropriately performed. In this embodiment, the determination that the second step has been appropriately performed means that a series of operations from the preparation of the sample from the fixed embedded tissue specimen to the acquisition of the measured value has been appropriately performed.

On the other hand, when the difference or the ratio between the measured value of the pseudo tissue and the predetermined reference value deviates from the predetermined numerical range, it may be determined that the second step has not been appropriately performed. In this embodiment, the determination that the acquisition of the measured value of the tissue specimen has not been appropriately performed means that all or a part of the series of operations from the preparation of the sample from the fixed embedded tissue specimen to the acquisition of the measured value has not been appropriately performed.

### [4. Kit for Accuracy Control of Specimen Analysis]

Disclosed but not claimed herein is a kit for accuracy control of specimen analysis (hereinafter also referred to simply as "kit") used for the above accuracy control method. The kit disclosed but not claimed herein includes the above fixed embedded pseudo tissue. The kit may further contain a reagent for dispersing a pseudo tissue into individual cells. Examples of such reagent include a solution of the above protease. In addition, the kit may further contain a reagent for detecting a predetermined molecular marker. Examples of such reagent include an antibody that specifically binds to the above predetermined molecular marker.

The fixed embedded pseudo tissue may be stored in a box and provided to the user. This box may include all of the fixed embedded pseudo tissue and the above reagents, or only a part thereof may be stored. In this box, a package insert that describes how to use fixed embedded pseudo tissues and the like may be further included together.

Disclosed but not claimed herein is the use of fixed embedded pseudo tissues in the accuracy control method. The accuracy control method and the fixed embedded pseudo tissue are as described above.

Hereinafter, the present disclosure will be described in more detail by examples, but the present disclosure is not limited to these examples.

### EXAMPLES

### Example 1: Preparation of Fixed Embedded Pseudo Tissue Using Two Types of Cells

### (1) Cells

MCF7 cells of a hormone receptor-positive breast cancer cell line and HCC1806 cells of a hormone receptor-negative breast cancer cell line were used. These cell lines were obtained from JCRB Cell Bank. The MCF7 cells express molecular markers such as estrogen receptor (ER) and progesterone receptor (PgR). The HCC1806 cells have strong intercellular adhesion properties.

### (2) Procedure for Preparation

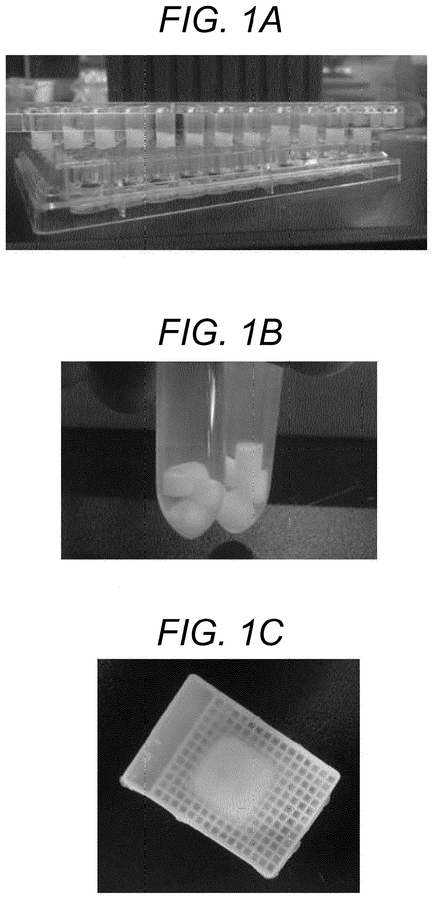

The MCF7 cells and the HCC1806 cells were cultured in a growth medium containing 10% fetal bovine serum (FBS) by a conventional method and recovered by trypsin treatment. The number of cells of the recovered MCF7 cells and HCC1806 cells was measured. The MCF7 cells and the HCC1806 cells were mixed at a ratio of the number of cells (MCF7 : HCC1806) of 1 : 1, 1 : 2, 1 : 3 or 1 : 4. The mixed cells were dispensed into each transwell insert of Transwell (registered trademark) 96-well plate (Corning Incorporated) and incubated for 1.5 hours in a growth medium containing 10% FBS. After gently centrifuging the plate, the medium on the cell mass and the medium under the transwell insert were removed, and the cell mass was washed once with PBS. Each well was filled with a 10% neutral buffered formalin solution, and the cell mass was fixed at room temperature for 24 hours (see Fig. 1A). After fixation, the formalin solution was removed and the cell mass was washed with PBS, and 70% ethanol was added to each well. The columnar cell mass was recovered from the transwell insert in a tube containing 70% ethanol to obtain a pseudo tissue (see Fig. 1B). The resulting pseudo tissue was dehydrated stepwise with a rising series of ethanol by a conventional method, and subjected to xylene treatment to perform paraffin penetration. The paraffin-penetrated pseudo tissue was embedded in paraffin by a conventional method to prepare an FFPE pseudo tissue (see Fig. 1C).

### Example 2: Preparation of Fixed Embedded Pseudo Tissue from One Type of Cells

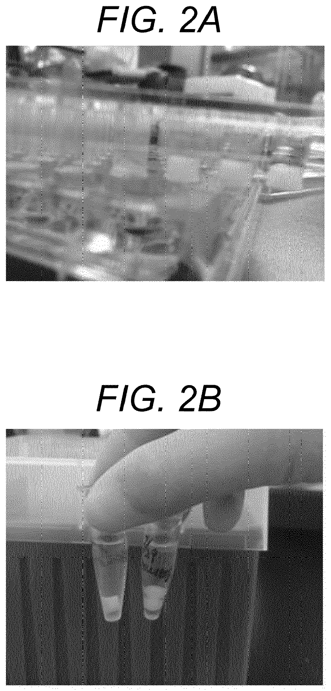

HCC1806 cells were dispensed into each transwell insert of Transwell (registered trademark) 96-well plate (Corning Incorporated) and incubated for 1.5 hours in growth medium containing 10% FBS. The cell mass was washed as in Example 1, each well was filled with a 10% neutral buffered formalin solution, and the cell mass was fixed at room temperature for 20 hours (see Fig. 2A). After fixation, the formalin solution was removed and the cell mass was washed with PBS, and 70% ethanol was added to each well. The columnar cell mass was recovered from the transwell insert in a tube containing 70% ethanol to obtain a pseudo tissue (see Fig. 2B). In the same manner as in Example 1, an FFPE pseudo tissue was prepared from the obtained pseudo tissue.

### Example 3: Observation and Evaluation of Pseudo Tissue

### (1) Observation of Thin sliced section of Pseudo Tissue

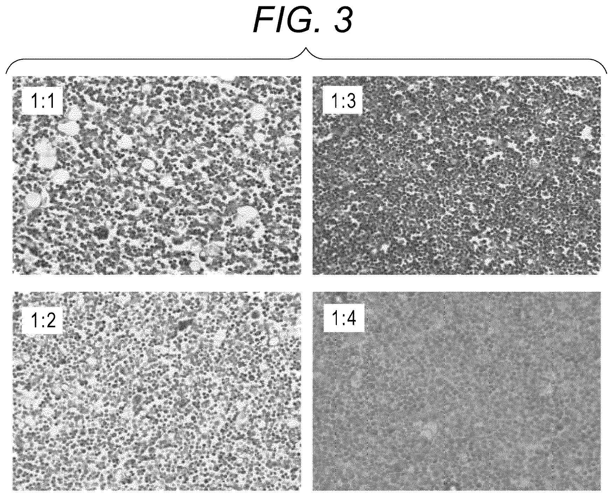

A thin sliced section was acquired from each FFPE pseudo tissue prepared in Example 1 and attached to a glass slide. The obtained thin sliced sections were stained with HE by a conventional method and observed with an optical microscope. The results are shown in Fig. 3. As can be seen from Fig. 3, the section of the pseudo tissue showed a tissue-like structure regardless of the mixing ratio.

### (2) Confirmation of Homogeneity

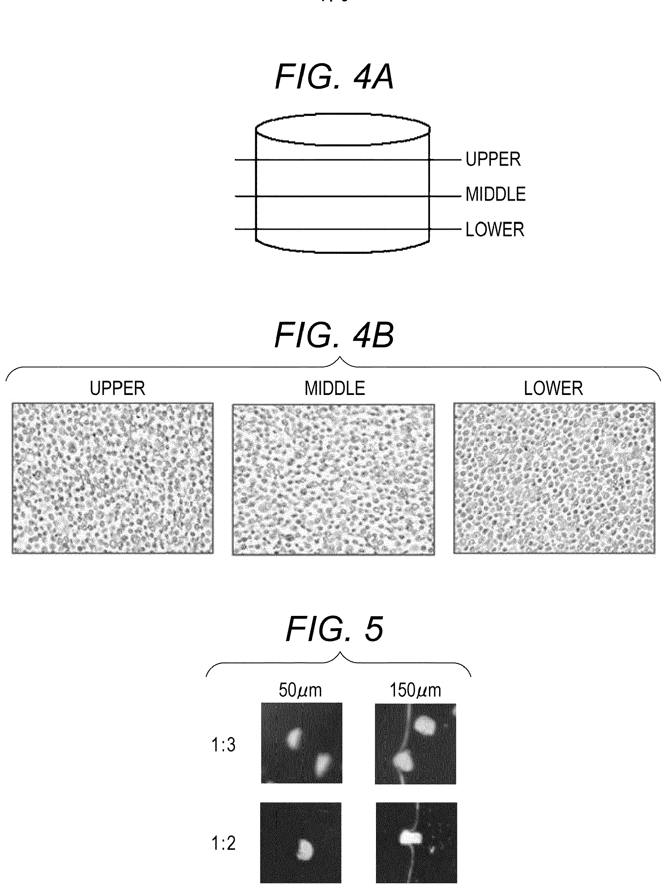

In the FFPE pseudo tissue prepared in Example 1, the columnar pseudo tissue is embedded in paraffin. In order to confirm that the pseudo tissue was substantially homogeneous, the FFPE pseudo tissue with a mixing ratio (MCF7 : HCC1806) of 1 : 2 was cut in the upper part, the middle part and the lower part (see Fig. 4A). The obtained thin sliced sections were stained with HE and observed with an optical microscope. The results are shown in Fig. 4B. As can be seen from Fig. 4B, a similar tissue-like structure was shown in the thin sliced section obtained from any site. That is, the MCF cells and the HCC1806 cells were substantially uniformly dispersed in the pseudo tissue. Thus, the pseudo tissue was shown to be substantially homogeneous.

### (3) Application of Deparaffinization Treatment

When the FFPE pseudo tissue prepared in Example 1 was deparaffinized to acquire a pseudo tissue, whether the pseudo tissue could maintain a tissue-like state was confirmed. Specific operations are as follows. Thin sliced sections with a thickness of 50 µm or 150 µm were obtained by cutting the FFPE pseudo tissues with mixing ratios (MCF7 : HCC1806) of 1 : 2 and 1:3. Each thin sliced section was placed in a tube, and deparaffinized by adding xylene thereto and standing for 10 minutes. The deparaffinized thin sliced sections were observed with the naked eyes to confirm whether or not the tissues were disintegrated. The results are shown in Fig. 5. In the upper left and upper right panels of Fig. 5, two thin sliced sections are shown. In the upper right and lower right panels of Fig. 5, the streaky line is a boundary of the liquid. As can be seen from Fig. 5, none of the thin sliced sections was disintegrated by deparaffinization treatment using xylene. Based on this, it was shown that the thin sliced sections of the pseudo tissue can be recovered by subjecting the thin sliced sections of the FFPE pseudo tissue to deparaffinization treatment using xylene.

### Example 4: Immunohistological Staining of Pseudo Tissue

Using the pseudo tissue obtained from the FFPE pseudo tissue prepared in Example 1 as a specimen, whether a molecular marker could be detected by immunohistological staining was confirmed. Molecular markers to be detected were ER and PgR expressed in the MCF7 cells. Specific operations are as follows.

### (1) Immunohistological Staining

The FFPE pseudo tissue with a mixing ratio (MCF7 : HCC1806) of 1 : 2 prepared in Example 1 was cut in the upper part, the middle part and the lower part to obtain thin sliced sections with a thickness of 50 µm. Each thin sliced section was attached to a glass slide and deparaffinized using xylene. The deparaffinized thin sliced section was re-hydrophilized with a descending series of ethanol. The thin sliced section was washed once with ultrapure water. The glass slide to which the thin sliced section was attached was placed in distilled water to which Immunosaver (registered trademark) (Nisshin EM Co., Ltd.) was added, and heated at 98°C for 20 minutes to perform antigen activation treatment. The thin sliced section was washed once with PBS. Immunohistological staining was performed on the thin sliced section by DAB method. A mouse anti-ER antibody or a mouse anti-PgR antibody was used as a primary antibody, and a peroxidase-labeled anti-mouse IgG antibody was used as a secondary antibody.

### (2) Results

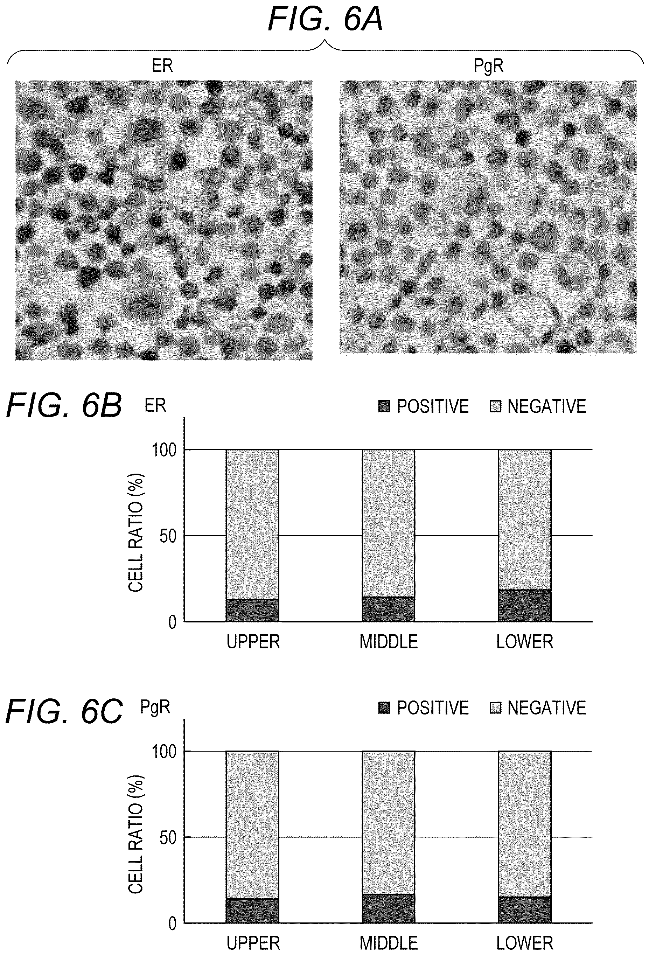

An example of the result of immunohistological staining is shown in Fig. 6A. The molecular marker-positive cells and the molecular marker-negative cells in each thin sliced section were counted with an optical microscope. The ratios of the molecular marker-positive cells to the molecular marker-negative cells are shown in Figs. 6B and 6C. As can be seen from Fig. 6A, the molecular markers (ER and PgR) expressed in the cells in the pseudo tissue could be detected by immunohistological staining of the pseudo tissue acquired from the FFPE pseudo tissue. It is considered that the molecular marker-positive cells are MCF7 cells derived from hormone positive breast cancer cell lines, and the molecular marker-negative cells are HCC1806 cells derived from hormone negative breast cancer cell lines. As shown in Figs. 6B and 6C, the ratios of the molecular marker-positive cells to the molecular marker-negative cells were similar, in the thin sliced section obtained from any site. Thus, it was also shown by immunohistological staining that the pseudo tissue is substantially homogeneous.

### Example 5: FCM Analysis of Cells Recovered from Pseudo Tissue

The pseudo tissue acquired from the FFPE pseudo tissue was dispersed by the enzyme to recover the cells. Using the obtained cells as specimens, whether they could be analyzed with a flow cytometer was confirmed. Specific operations are as follows.

### (1) Cell Recovery

The FFPE pseudo tissue with a mixing ratio (MCF7 : HCC1806) of 1 : 2 prepared in Example 1 was cut to acquire a thin sliced section with a thickness of 50 µm. The thin sliced section was placed in a tube, and deparaffinized by adding xylene thereto. The deparaffinized thin sliced section was re-hydrophilized with a descending series of ethanol. The thin sliced section was washed once with ultrapure water. Immunosaver (registered trademark) (Nisshin EM Co., Ltd.) diluted with distilled water was added to the tube containing the thin sliced section, and the tube was heated at 98°C for 20 minutes to perform antigen activation treatment. The thin sliced section was washed once with PBS. A solution (200 µL) of Dispase I + II (product number 17105041, GIBCO) or Dispase II (product number 383-02281, Wako Pure Chemical Industries, Ltd.) was added to the tube containing the thin sliced section at various enzyme concentrations, and the mixture was subjected to enzyme treatment at 37°C for 10 minutes. The enzyme concentration of the enzyme solution added to the tube is 1.6, 3.2, 6.4 or 12.8 U/mL for Dispase I + II, and 1000 or 2000 U/mL for Dispase II. The enzyme was deactivated by adding a 1 mM EDTA/0.5% BSA/PBS solution to the tube. By the action of the enzyme, the thin sliced section of the pseudo tissue was dispersed into individual cells. The obtained cells were washed three times with a 0.5% BSA/PBS solution.

### (2) FCM Analysis

In Example 5, Ki-67, a proliferation marker of breast cancer, was detected as a molecular marker to be detected. The cells obtained above were immunostained with an anti-Ki-67 antibody (clone name Ki-S5, MAB4190, Merck Millipore) and a fluorescently labeled anti-mouse IgG antibody (secondary antibody). Cell nuclei were stained with DAPI. As a negative control, cells reacted with only the secondary antibody were also prepared. The stained cells were used as a sample to be subjected to FCM analysis. Standard beads for counting the number of cells (50 µL) (CountBright (trademark) Absolute Counting Beads, C36950, Molecular Probes) was added to the total amount of the sample, and the sample was measured with an imaging flow cytometer (BD biosciences). The standard beads are fluorescently labeled, and the number of beads per 50 µL is known. By measuring the cell samples to which the standard beads are added, the cell concentrations in the cell samples can be calculated from the number of events of the standard beads and the number of events of the cells. According to the manual attached to the standard beads, the cell concentrations (cells/µL) of the samples containing the stained cells were acquired.

### (3) Results

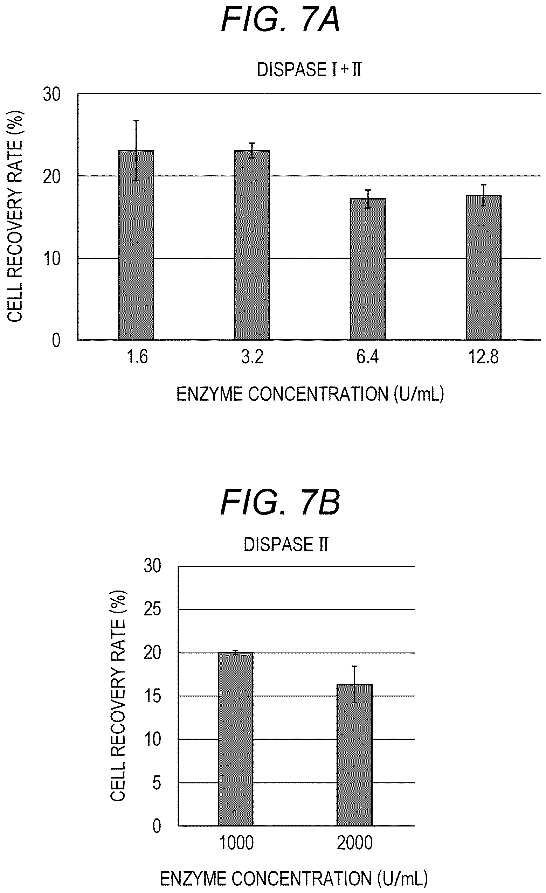

The number of cells of the FFPE pseudo tissue prepared in Example 1 is known, and from this number, the number of cells contained in the thin sliced section of the (1) above is calculated to be 4.8 × 10⁵ cells. From this value and the cell concentrations acquired in the (2) above, the cell recovery rates from the FFPE pseudo tissue were calculated. The results are shown in Figs. 7A and 7B. As shown in these figures, it was found that the ratios (recovery rates) of cells acquired from the FFPE pseudo tissue by the pretreatment step of FCM analysis such as deparaffinization treatment and cell dispersion treatment are about 20%.

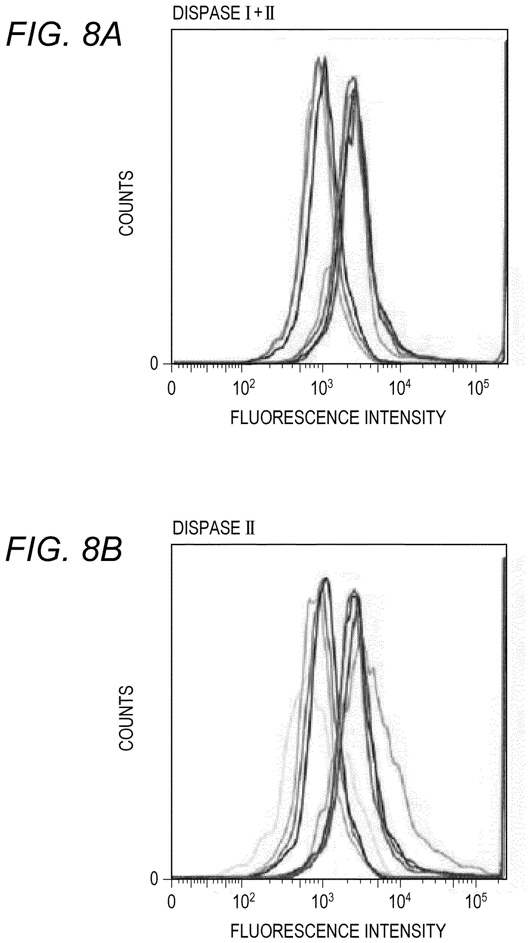

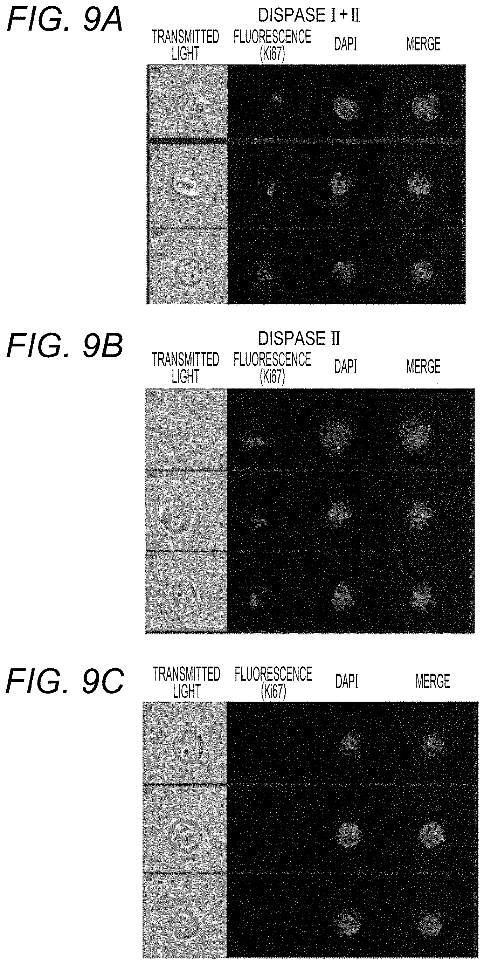

As detection results of molecular markers by FCM analysis, histograms shown in Figs. 8A and 8B were obtained. An example of images of cells taken by an imaging flow cytometer is shown in Figs. 9A to 9C. In Figs. 8A and 8B, the left peak shows the negative control reacted only with the secondary antibody, and the right peak shows the sample immunostained with the anti-Ki-67 antibody and the secondary antibody. Thus, it was found that the immunostained samples and the negative control can be clearly distinguished. As shown in Figs. 9A and 9B, expression of Ki-67 was observed in the images of the cells immunostained with the anti-Ki-67 antibody. However, as shown in Fig. 9C, expression of Ki-67 was not observed in the images of the negative control reacted with only the secondary antibody. From these facts, it was shown that the cells recovered from the FFPE pseudo tissues can be detected by FCM analysis by immunostaining molecular markers. No decrease in dyeability due to increase in the concentration of the cell dispersing enzyme was observed.

From the above, it is possible to control the accuracy of the steps from acquisition of cells from FFPE specimens to FCM analysis by using the FFPE pseudo tissue as a positive control. In addition, the FFPE pseudo tissue prepared by a cell line not expressing a molecule marker to be detected can also be used as a negative control. Accuracy control can be performed by determining the success or failure of FCM analysis in which cells are dispersed from tissue specimens based on the results of controls using these pseudo tissues.

### Reference Example: Confirmation of Expression of Cell Adhesion Molecule

### (1) Confirmation by Western Blot

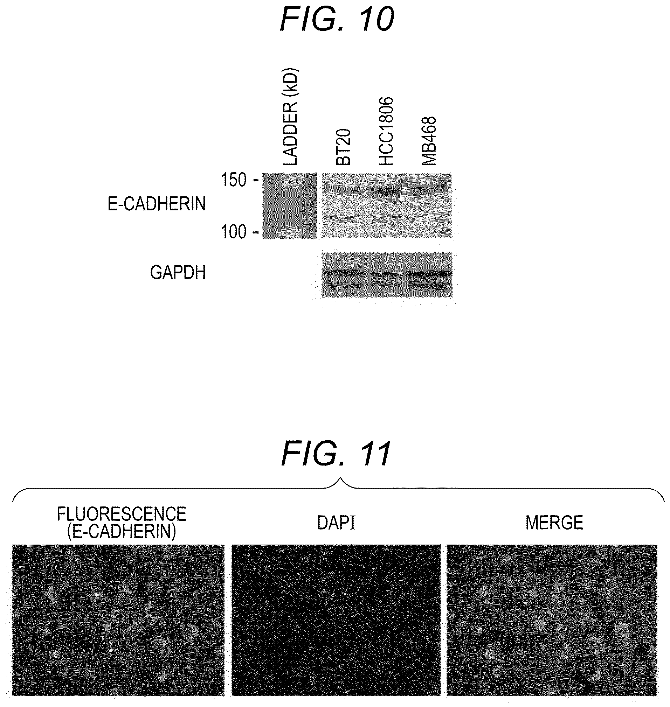

BT20, HCC1806 and MB468 cells were used as cell lines. These cells were solubilized with RIPA buffer (containing 0.5 w/v% sodium deoxycholate, 0.1 w/v% sodium dodecyl sulfate, 1.0 w/v% NP-40 substitute), and a supernatant was obtained by centrifugation. A sample buffer was added to the obtained supernatant, and the mixture was boiled to prepare a sample for SDS-PAGE. The obtained sample was separated by SDS-PAGE according to an ordinary method, and membrane transfer was performed. Western blotting using an anti-E-cadherin antibody and an anti-GAPDH antibody was performed on the obtained membrane, and expression of cadherin was confirmed. The results are shown in Fig. 10. As can be seen from Fig. 10, it could be confirmed that E-cadherin was expressed in the BT20, HCC1806 and MB468 cells.

### (2) Confirmation by Fluorescence Microscope

The FFPE pseudo tissue with a mixing ratio (MCF7 : HCC1806) of 1 : 2 prepared in Example 1 was cut to acquire a thin sliced section with a thickness of 4 µm. In the same manner as in Example 4, acquisition of a pseudo tissue from the thin sliced section and antigen activation treatment were performed. The resulting pseudo tissue was immunohistologically stained with an anti-E-cadherin antibody and a fluorescently labeled anti-mouse IgG antibody. Cell nuclei were stained with DAPI. The stained pseudo tissue was observed with a fluorescence microscope BZ-9000 (KEYENCE CORPORATION), and expression of cadherin was confirmed. The results are shown in Fig. 11. As can be seen from Fig. 11, it could be confirmed that E-cadherin was expressed in the HCC1806 cells in the pseudo tissue.

## Claims

1. A method for controlling accuracy of specimen analysis including
a first step of dispersing the cells of the fixed embedded pseudo tissue comprising a pseudo tissue comprising a plurality of cells derived from a cell line and a cell adhesion molecule produced by the cells, wherein the cells are adhered by the cell adhesion molecule in the pseudo tissue, into individual cells to prepare a control sample, and obtaining a measured value representing the number of cells by flow cytometer,
a second step of dispersing cells in a fixed embedded tissue specimen into individual cells to prepare a measurement sample, and obtaining a measured value representing the number of cells by flow cytometer, and
a step of comparing the measured value of the control sample with a predetermined reference value and determining whether or not the second step has been appropriately performed based on the comparison result.

2. The method according to claim 1, further obtaining a measured value representing the number of molecular marker-positive cells, in the measurement step by the flow cytometer.

3. The method according to claim 1 or 2, wherein,
in the determination step, when a difference or a ratio between the measured value of the control sample and the predetermined reference value is within a predetermined numerical range, it is determined that the second step has been appropriately performed, and
when the difference or the ratio between the measured value of the control sample and the predetermined reference value deviates from the predetermined numerical range, it is determined that the second step has not been appropriately performed.

4. The method according to any one of claims 1 to 3, wherein,
the first step comprises: acquiring a pseudo tissue from the fixed embedded pseudo tissue; contacting a protease with the pseudo tissue; and degrading the cell adhesion molecule in the pseudo tissue whereby the cells of the pseudo tissue are dispersed into individual cells, and
the second step comprises: acquiring a tissue specimen from the fixed embedded tissue specimen; contacting a protease of the same type as the protease with the tissue specimen; and degrading the cell adhesion molecule in the tissue specimen whereby the cells of the tissue specimen are dispersed into individual cells.

5. A method for controlling accuracy of specimen analysis including
a first step of acquiring a pseudo tissue from the fixed embedded pseudo tissue comprising a pseudo tissue comprising a plurality of cells derived from a cell line and a cell adhesion molecule produced by the cells, wherein the cells are adhered by the cell adhesion molecule in the pseudo tissue, and obtaining a measured value representing an expression level of a molecular marker or the number of molecular marker-positive cells, from the pseudo tissue, and
a step of comparing the measured value with a predetermined reference value and determining whether or not the first step has been appropriately performed based on the comparison result.

6. The method according to claim 5, wherein the step of obtaining the measured value is performed by flow cytometer or a microscope.

7. The method according to claim 5 or 6, wherein,
in the determination step, when a difference or a ratio between the measured value of the pseudo tissue and the predetermined reference value is within a predetermined numerical range, it is determined that the first step has been appropriately performed, and
when the difference or the ratio between the measured value of the pseudo tissue and the predetermined reference value deviates from the predetermined numerical range, it is determined that the first step has not been appropriately performed.

8. The method according to any one of claims 5 to 7, further comprising a second step of acquiring the tissue specimen from a fixed embedded tissue specimen, and obtaining a measured value representing an expression level of a molecular marker or the number of molecular marker-positive cells, from the tissue specimen.

## Patentansprüche

1. Verfahren zur Kontrolle einer Genauigkeit einer Munsteranalyse, Folgendes umfassend
einen ersten Schritt des Dispergierens der Zellen des fest eingebetteten Pseudogewebes, das ein Pseudogewebe umfasst, das mehrere Zellen, die von einer Zelllinie abgeleitet sind, und ein Zellanhaftungsmolekül umfasst, das durch die Zellen hergestellt wird, wobei die Zellen durch das Zellanhaftungsmolekül in dem Pseudogewebe anhaften, in individuelle Zellen, um eine Kontrollprobe herzustellen, und des Ermittelns eines gemessenen Werts, der die Anzahl Zellen repräsentiert, durch ein Durchflusszytometer,
einen zweiten Schritt des Dispergierens von Zellen in einer fest eingebetteten Gewebemunster in individuelle Zellen, um eine Messprobe herzustellen, und des Ermittelns eines gemessenen Werts, der die Anzahl Zellen repräsentiert, durch ein Durchflusszytometer, und
einen Schritt des Vergleichens des gemessenen Werts der Kontrollprobe mit einem vorgegebenen Referenzwert und des Bestimmens, ob der zweite Schritt sachgerecht durchgeführt wurde oder nicht, auf der Grundlage des Vergleichsergebnisses.

2. Verfahren nach Anspruch 1, weiterhin Ermitteln eines gemessenen Werts, der die Anzahl Molekularmarker-positiver Zellen repräsentiert, in dem Messschritt durch das Durchflusszytometer umfassend.

3. Verfahren nach Anspruch 1 oder 2, wobei
in dem Bestimmungsschritt, wenn eine Differenz oder ein Verhältnis zwischen dem gemessenen Wert der Kontrollprobe und dem vorgegebenen Referenzwert innerhalb eines vorgegebenen numerischen Bereichs ist, festgestellt wird, dass der zweite Schritt sachgerecht durchgeführt wurde, und
wenn die Differenz oder das Verhältnis zwischen dem gemessenen Wert der Kontrollprobe und dem vorgegebenen Referenzwert von einem vorgegebenen numerischen Bereich abweicht, festgestellt wird, dass der zweite Schritt nicht sachgerecht durchgeführt wurde.

4. Verfahren nach einem der Ansprüche 1 bis 3, wobei
der erste Schritt Folgendes umfasst: Erlangen eines Pseudogewebes aus dem festen eingebetteten Pseudogewebe; Kontaktieren einer Protease mit dem Pseudogewebe; und Abbauen des Zellanhaftungsmoleküls in dem Pseudogewebe, wodurch die Zellen des Pseudogewebes in individuelle Zellen dispergiert werden, und
der zweite Schritt Folgendes umfasst: Erlangen einer Gewebemunster aus der festen eingebetteten Gewebemunster; Kontaktieren einer Protease des gleichen Typs wie die Protease mit der Gewebemunster; und Abbauen des Zellanhaftungsmoleküls in der Gewebemunster, wodurch die Zellen der Gewebemunster in individuelle Zellen dispergiert werden.

5. Verfahren zur Kontrolle einer Genauigkeit einer Munsteranalyse, Folgendes umfassend
einen ersten Schritt des Erlangens eines Pseudogewebes aus dem fest eingebetteten Pseudogewebe, das ein Pseudogewebe umfasst, das mehrere Zellen, die von einer Zelllinie abgeleitet sind, und ein Zellanhaftungsmolekül umfasst, das durch die Zellen hergestellt wird, wobei die Zellen durch das Zellanhaftungsmolekül in dem Pseudogewebe anhaften, und Ermitteln eines gemessenen Werts, der einen Expressionsgrad eines molekularen Markers oder die Anzahl Molekularmarker-positiver Zellen repräsentiert, aus dem Pseudogewebe, und
einen Schritt des Vergleichens des gemessenen Werts mit einem vorgegebenen Referenzwert und des Bestimmens, ob der erste Schritt sachgerecht durchgeführt wurde oder nicht, auf der Grundlage des Vergleichsergebnisses.

6. Verfahren nach Anspruch 5, wobei der Schritt des Ermittelns des gemessenen Werts mit einem Durchflusszytometer oder mit einem Mikroskop durchgeführt wird.

7. Verfahren nach Anspruch 5 oder 6, wobei
in dem Bestimmungsschritt, wenn eine Differenz oder ein Verhältnis zwischen dem gemessenen Wert des Pseudogewebes und dem vorgegebenen Referenzwert innerhalb eines vorgegebenen numerischen Bereichs ist, festgestellt wird, dass der erste Schritt sachgerecht durchgeführt wurde, und
wenn die Differenz oder das Verhältnis zwischen dem gemessenen Wert des Pseudogewebes und dem vorgegebenen Referenzwert von einem vorgegebenen numerischen Bereich abweicht, festgestellt wird, dass der erste Schritt nicht sachgerecht durchgeführt wurde.

8. Verfahren nach einem der Ansprüche 5 bis 7, weiterhin einen zweiten Schritt des Erlangens der Gewebemunster aus einer festen eingebetteten Gewebemunster und des Ermittelns eines gemessenen Werts, der einen Expressionsgrad eines molekularen Markers oder die Anzahl Molekularmarker-positiver Zellen repräsentiert, aus der Gewebemunster umfassend.

## Revendications

1. Procédé destiné à contrôler la précision d'une analyse d'échantillon comportant
une première étape de dispersion des cellules du pseudo-tissu fixé enrobé comprenant un pseudo-tissu comprenant une pluralité de cellules dérivées d'une lignée cellulaire et une molécule d'adhésion cellulaire produite par les cellules, les cellules sont adhérées par la molécule d'adhésion cellulaire dans le pseudo-tissu, en cellules individuelles pour préparer un échantillon témoin, et obtention d'une valeur mesurée représentant le nombre de cellules par un cytomètre en flux,
une deuxième étape de dispersion de cellules dans un échantillon de tissu fixé enrobé en cellules individuelles pour préparer un échantillon de mesure, et obtention d'une valeur mesurée représentant le nombre de cellules par un cytomètre en flux, et
une étape de comparaison de la valeur mesurée de l'échantillon témoin avec une valeur de référence prédéterminée et détermination que la deuxième étape a ou n'a pas été adéquatement effectuée sur la base du résultat de comparaison.

2. Procédé selon la revendication 1, comprenant en outre l'obtention d'une valeur mesurée représentant le nombre de cellules positives à un marqueur moléculaire, à l'étape de mesure par le cytomètre en flux.

3. Procédé selon la revendication 1 ou 2, dans lequel,
à l'étape de détermination, quand une différence ou un rapport entre la valeur mesurée de l'échantillon témoin et la valeur de référence prédéterminée se situe à l'intérieur d'une gamme numérique prédéterminée, il est déterminé que la deuxième étape a été adéquatement effectuée, et
quand la différence ou le rapport entre la valeur mesurée de l'échantillon témoin et la valeur de référence prédéterminée s'écarte de la gamme numérique prédéterminée, il est déterminé que la deuxième étape n'a pas été adéquatement effectuée.

4. Procédé selon l'une quelconque des revendications 1 à 3, dans lequel
la première étape comprend : l'acquisition d'un pseudo-tissu à partir du pseudo-tissu fixé enrobé ; la mise en contact d'une protéase avec le pseudo-tissu ; et la dégradation de la molécule d'adhésion cellulaire dans le pseudo-tissu, moyennant quoi les cellules du pseudo-tissu sont dispersées en cellules individuelles, et
la deuxième étape comprend : l'acquisition d'un échantillon de tissu à partir de l'échantillon de tissu fixé enrobé ; la mise en contact d'une protéase du même type que la protéase avec l'échantillon de tissu ; et la dégradation de la molécule d'adhésion cellulaire dans l'échantillon de tissu, moyennant quoi les cellules de l'échantillon de tissu sont dispersées en cellules individuelles.

5. Procédé destiné à contrôler la précision d'une analyse d'échantillon comportant
une première étape d'acquisition d'un pseudo-tissu à partir du pseudo-tissu fixé enrobé comprenant un pseudo-tissu comprenant une pluralité de cellules dérivées d'une lignée cellulaire et une molécule d'adhésion cellulaire produite par les cellules, les cellules sont adhérées par la molécule d'adhésion cellulaire dans le pseudo-tissu, et obtention d'une valeur mesurée représentant un niveau d'expression d'un marqueur moléculaire ou le nombre de cellules positives à un marqueur moléculaire, à partir du pseudo-tissu, et
une étape de comparaison de la valeur mesurée avec une valeur de référence prédéterminée et détermination que la première étape a ou n'a pas été adéquatement effectuée sur la base du résultat de comparaison.

6. Procédé selon la revendication 5, dans lequel l'étape d'obtention de la valeur mesurée est effectuée par un cytomètre en flux ou un microscope.

7. Procédé selon la revendication 5 ou 6, dans lequel,
à l'étape de détermination, quand une différence ou un rapport entre la valeur mesurée du pseudo-tissu et la valeur de référence prédéterminée se situe à l'intérieur d'une gamme numérique prédéterminée, il est déterminé que la première étape a été adéquatement effectuée, et
quand la différence ou le rapport entre la valeur mesurée du pseudo-tissu et la valeur de référence prédéterminée s'écarte de la gamme numérique prédéterminée, il est déterminé que la première étape n'a pas été adéquatement effectuée.

8. Procédé selon l'une quelconque des revendications 5 à 7, comprenant en outre une deuxième étape d'acquisition de l'échantillon de tissu à partir d'un échantillon de tissu fixé enrobé, et obtention d'une valeur mesurée représentant un niveau d'expression d'un marqueur moléculaire ou le nombre de cellules positives à un marqueur moléculaire, à partir de l'échantillon de tissu.
